# EUROPEAN PATENT SPECIFICATION

(11) **EP 1 466 648 B1**
(45) Date of publication and mention of the grant of the patent: **06.04.2011**
(21) Application number: 02785032.0
(22) Date of filing: 16.12.2002
(51) Int. Cl.: A61N 7/00

(54) **ULTRASONIC TREATMENT DEVICE**
ULTRASCHALLBEHANDLUNGSVORRICHTUNG
DISPOSITIF THERAPEUTIQUE A ULTRASONS

(30) Priority: 17.12.2001 CN 01144259; 17.12.2001 CN 01278379 U
(43) Date of publication of application: 13.10.2004
(73) Proprietor: CHONGQING HAIFU(HIFU)TECHNOLOGY CO., LTD, Chongqing 401121 (CN)
(72) Inventor: ZHAO, Chunliang c/o Chongqing Haifu(HIFU) Technology Co., LTD., Chongqing 401121 (CN); WANG, Zhibiao c/o Chongqing Haifu(HIFU) Technology Co., LTD., Chongqing 401121 (CN); WANG, Zhilong c/o Chongqing Haifu(HIFU) Technology Co., LTD., Chongqing 401121 (CN); YE, Fangwei c/o Chongqing Haifu(HIFU) Technology Co., LTD., Chongqing 401121 (CN)
(74) Representative: Graf Lambsdorff, Matthias
(86) International application number: PCT/CN2002/000892
(87) International publication number: WO 2003/053521

(56) References cited:
- WO-A-00/36981
- WO-A-99/22652
- CN-A- 1 081 918
- DE-A1- 2 902 331
- US-A- 5 762 066
- US-B1- 6 206 843
- US-B1- 6 210 425
- US-B1- 6 217 530

## Description

### FIELD OF THE INVENTION

The present invention generally relates to the field of ultrasound treatment. More particularly, the present invention relates to an ultrasound therapeutic equipment useful for treating skin lesions of gynecology and other human being's skin lesions with focused ultrasound.

### BACKGROUD OF THE INVENTION

The ultrasound therapy as a surgical way for the diseased tissue has been developed in recent years. In principle, the focused ultrasound is used to destroy and necrotize the diseased tissue, and then the tissue necrosed is gradually absorbed by normal tissue, or the focused ultrasound exposure is deployed to bring benign changes to the diseased tissue. Prior to the present invention, the techniques to bring the high-intensity focused ultrasound have been well developed. However, the problem encountered by the medical circle and medical equipment industry is how to effectively practice the therapeutic ultrasound in clinic for treatment of various diseases on different part of patient.

The existing ultrasound therapeutic apparatus are mostly designed for tumor treatment of human being. Generally, the tumors are concentrated and located within tissue far from the ultrasound applicator, which supplies the therapeutic ultrasound, and they are_easily to be positioned and exposed by focused ultrasound from the ultrasound applicator. For this kind of ultrasound therapeutic devices treating tumors, the ultrasound applicator is required to be relatively fixed on a motion device, so that the ultrasound applicator can be driven by the mechanics aiming at the tumor. Furthermore, a high-intensity ultrasound exposure and an ultrasound applicator of a big size are required to destroy the tumor.

The feature of the skin lesions including the skin lesions of gynecology is different from that of tumors. The skin lesions are distributed on skin surface or under subcutaneous tissue, close to ultrasound applicator. Also, the skin lesions are small and sensitive to ultrasound exposure. Therefore, it is unpractical to use a big and heavy ultrasound applicator of the prior techniques to locate and treat the skin lesions. It is unsuitable to use the ultrasound applicator, which is relatively fixed on a motion device to treat the skin lesions, which are, in most cases, distributed in a wide area. Additionally, a small intensity of ultrasound is applied to destroying the skin lesions and the ultrasound energy is required to be attenuated rapidly after the ultrasound beam going through the epidermal tissue and lower subcutaneous tissue in order to avoid destroying the deeper tissue within body. Therefore, an ultrasound therapeutic apparatus with a high frequency dedicated for treating skin lesions is needed in clinic.

A handheld ultrasound treatment device is disclosed in DE 2902 331 A1 and US 5, 762, 066.

### SUMMARY OF THE INVENTION

In general, in one aspect, an object of the invention is to provide an equipment for treating skin lesions of a human being including the skin lesions of gynecology with a focused ultrasound.

Further, an object of the invention is to provide an equipment which is more convenient for clinicians to use in clinic to treat skin lesions of a human being including the skin lesions of gynecology with a focused ultrasound.

A further object of the invention is to provide an equipment with an ultrasound applicator which can be held by hand and is more convenient for clinicians to use in clinic to treat skin lesions of human being including the skin lesions of gynecology with a focused ultrasound.

A further object of the invention is to provide an equipment with a light and portable ultrasound applicator which is more convenient for clinicians to use in clinic to treat skin lesions of human being including the skin lesions of gynecology with a focused ultrasound.

A further object of the invention is to provide an equipment with an ultrasound applicator with a small front distal which is more convenient for clinicians to use in clinic to treat skin lesions of human being including the skin lesions of gynecology with a focused ultrasound.

A further object of the invention is to provide an ultrasound therapeutic equipment with a frequency width suitable for effectively treating skin lesions of a human being.

A further object of the invention is to provide an ultrasound therapeutic equipment with an acoustic power intensity suitable for effectively treating skin lesions of a human being.

A further object of the invention is to provide an ultrasound therapeutic equipment with a cooling means during treating skin lesions of a human being.

The invention is defined in appended claim 1. Prefered embodiments are described in the dependent claims.

The invention provides an ultrasound therapeutic equipment for treating skin lesions of a human being. The apparatus includes a controlling device, an ultrasound transducer transmitting the therapeutic ultrasound beam to a target region of the human being, and a focusing means. Said controlling device is a control system used to control the operation of the ultrasound therapeutic equipment. Said ultrasound transducer and said focusing means are installed in an ultrasound applicator which can be held by hand.

Said ultrasound applicator, which can be held by hand, includes a handle and a shell forming a lumen wherein said ultrasound transducer and said focusing means are placed.

Said controlling device has a frequency width ranging from 5MHz to 25MHz, and preferably, from 9 MHz to 11MHz.
The ultrasound applicator is capable of producing a focused ultrasound wave. The focal length of the ultrasound applicator ranges from 5mm to 50mm, and preferably, ranges from 9mm to 11mm. The acoustic output of the ultrasound applicator is more than 3W.

Said ultrasound therapeutic equipment of the present invention further includes a cooling means. Said cooling means includes a water tank connecting to a water piping system comprising of an inlet pipe and an outlet pipe, which is going through the ultrasound applicator suitable for being held by a hand. The water may flow into the lumen through the inlet water pipe and flow out of the lumen of the ultrasound applicator through the outlet water pipe so that the water tank, the inlet water pipe and outlet water pipe form a loop.

The diameter of the front distal of said ultrasound applicator ranges from 5mm to 80mm, and preferably, ranges from 14mm to 16mm.

Said ultrasound transducer is detachable and installed within the handheld ultrasound applicator.

Preferably, the water contained in the said cooling means is distilled water.

When the ultrasound therapeutic equipment of the present invention is used in clinic for treating skin lesions, the diseased part is exposed by the focused ultrasound beam with said appropriate parameters as specified in present patent document. The tissue cells in the diseased part will be oscillated and heated under the ultrasound exposure. When the ultrasound energy is high enough, the heat produced by focused ultrasound wave will deposit in the targeted tissue fast and the temperature of the targeted tissue will rise rapidly, e.g. in several seconds. The tissue edema by ultrasound radiation will be caused and the microcirculation in tissue is improved. The area for tissue edema can be controlled through appropriately controlling the parameters adopted by the ultrasound therapeutic equipment.

Animal experiments have been performed to verify the therapeutic effect of the therapy by using the ultrasound therapeutic equipment of the present invention. 160 New Zealand rabbits and 5 mini-swines were used in the animal experiments. The parameters specified in this patent application document were adopted in the experiments. After these animal experiments, the changes on the treated diseased part were observed and evaluated. The results of these experiments showed that the ultrasound wave could go through the skin surface and effect the subcutaneous tissue without causing damage to epidermis, the edema in subcutaneous tissue disappeared soon upon ultrasound exposure and the subcutaneous tissue recovered.

The vulvar dystrophy is a kind of chronic disease, characterizing in degeneration of pigments in skin, whitening of mucosa on vulvae, disorder growth of epidermis tissue and denaturalization of a hypodermis tissue. The vulvar dystrophy is a common gynecology disease of which the pathogeny is not quite clear. It is also a clinically refractory disease for which no specifically effective medicine has been identified. The equipment of the present invention was used to treat at least tens of persons suffering from vulvar dystrophy. During the treatment process, the patients had no bad reaction and didn't feel any discomfort. After the treatment, the edema in the treated region appeared and then disappeared within 3 days. Then the symptoms of vulvar dystrophy were relieved greatly, the pruritus faded away and the diseased part recovered normally. These treatment results showed that it is advantageous to use said ultrasound therapeutic equipment of the present invention to treat skin lesions instead of using other methods.

These and other advantages of the invention, along with the invention itself, will be more understood after a review of the description, figures for the description, and the appended claims.

### BRIEF DESCRIPTION OF THE FIGURES

FIG. 1 is a structural diagram of an ultrasound therapeutic equipment of the present invention.
FIG. 2 is a sectional diagram of interior structure of the handheld ultrasound applicator of the equipment of the present invention.
FIG. 3 is a block diagram of the controlling device of the equipment of the present invention.
FIG. 4 is a circuit diagram of the controlling device of the equipment of the present invention.
FIG. 5 is a circuit diagram of the water pump of the equipment of the present invention.

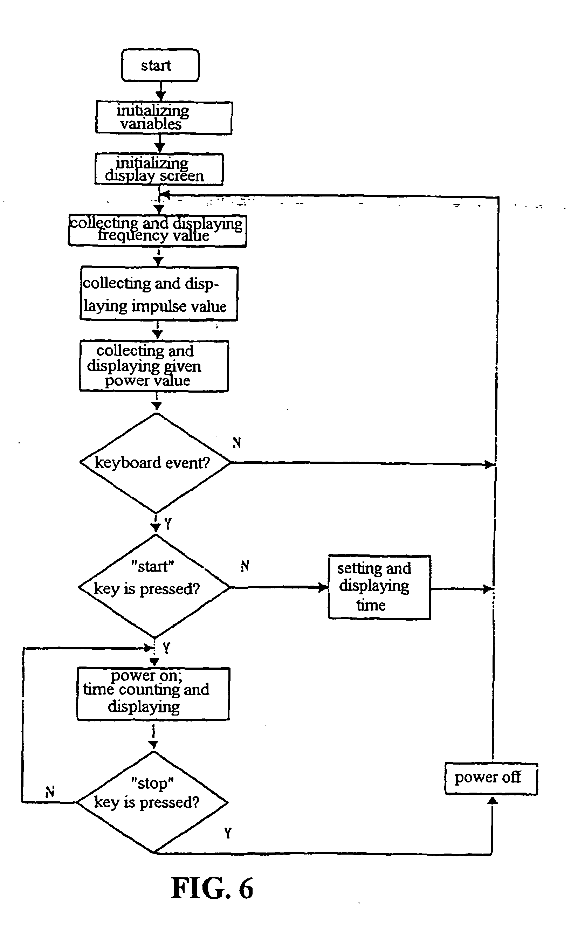

FIG. 6 is a flow diagram of showing the operation process of the controlling device of the equipment of the present invention.

### DETAILED DESCRIPTION OF PREFERRED EMBODIMENTS

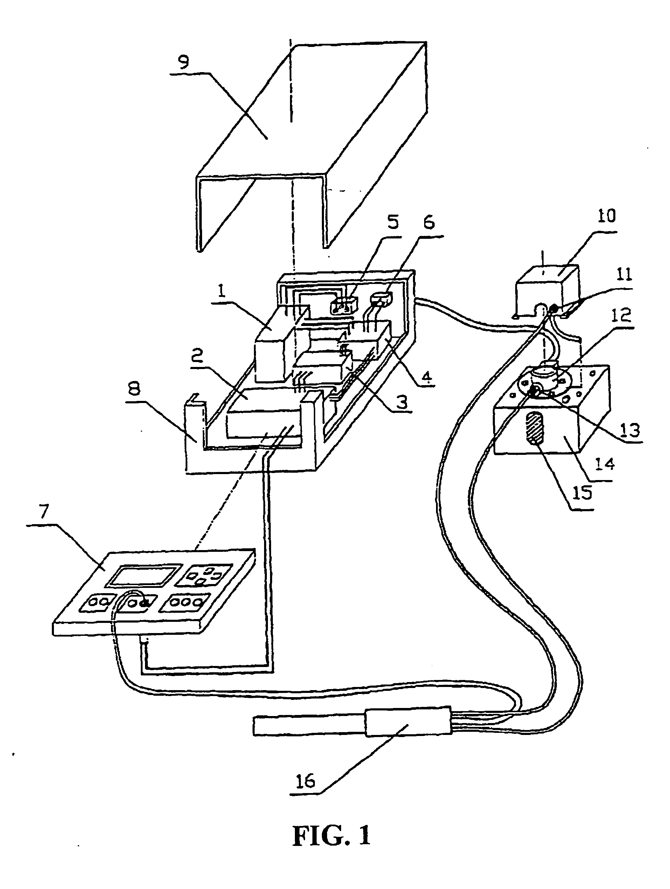

Referring to FIG. 1, the ultrasound therapeutic equipment includes a controlling device, an ultrasound applicator which can be held by hand, a cooling means and a panel. The controlling device contains a transformer, an electrical socket, a controller and a power amplifier. The power supply is connected to the transformer. The power supply is connected to the controller and then the controller is connected to the power amplifier. The controlling device and the ultrasound applicator which can be held by hand are both connected to the control panel. A display circuit is in the panel. The cooling means comprises a cooling tank which is connected to a circulating pump. The circulating pump is connected to the controlling device through a cable. The water outlet of cooling water tank is connected to the water inlet of the handheld ultrasound applicator through an inlet water pipe and the water outlet of the handheld ultrasound applicator is connected to the water inlet of the cooling water tank through an outlet water pipe.

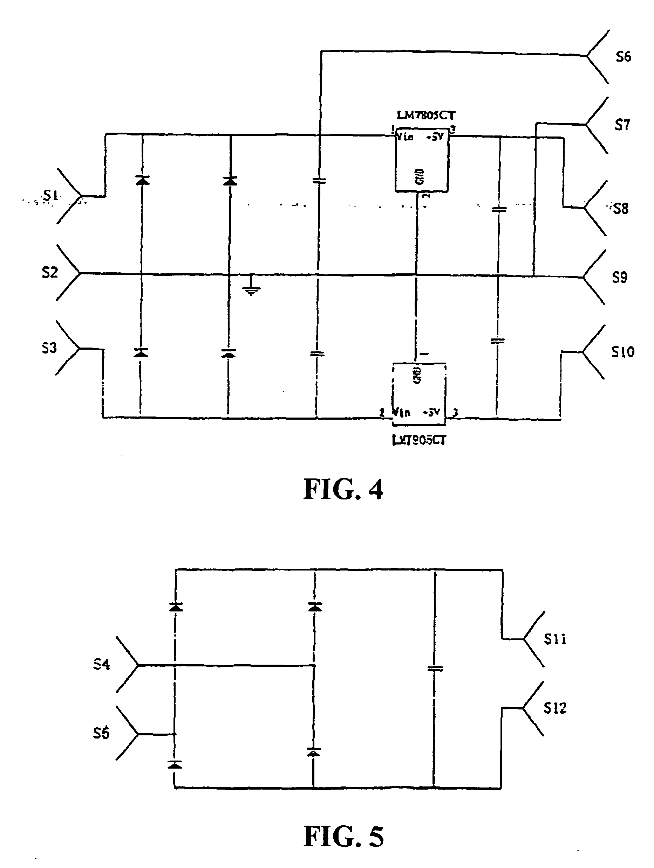

An external power supply is connected to a transformer 1 via an electrical socket 5. The transformer 1 is contained in the controlling device. The secondary winding of the transformer is connected to the power supply 4. The power supply 4 transfers the alternating current into a direct current and then supplies power respectively to a power amplifier 2 and a controller 3. Also, a direct current to the electrical socket 6 provided by the power supply is used to drive circulating water pump 12 in the cooling water tank. The rectifier in the power supply 4 consists of a bridge rectifying circuit and a voltage regulator. FIG. 4 their interior circuits. The controller 3 includes a singlechip and other digital integrate circuits, of which schematic diagram is shown in Figure 5. The panel is numbered as 7, the controlling device cabinet is numbered as 8 and the cover for the cabinet is numbered as 9. The power switches, adjusting knobs and output sockets on the console 7 are the standard components. Appropriate standard components can be determined and selected by a person skilled in the art. No specific limitations are set on the types of those standard components.

The cooling water tank 14 is made of a material, which is rustproof and moistureproof. And the water level in the cooling water tank 14 can be observed through a widow 15. The circulating water pump 12 pumps out the cooling water from the water outlet 13 to the water inlet of the handheld ultrasound applicator 16, and the water goes through the handheld ultrasound applicator 16 and then returns to the water inlet 11. The water tank cover is numbered as 10 and fixed on the cooling water tank 14 by screws.

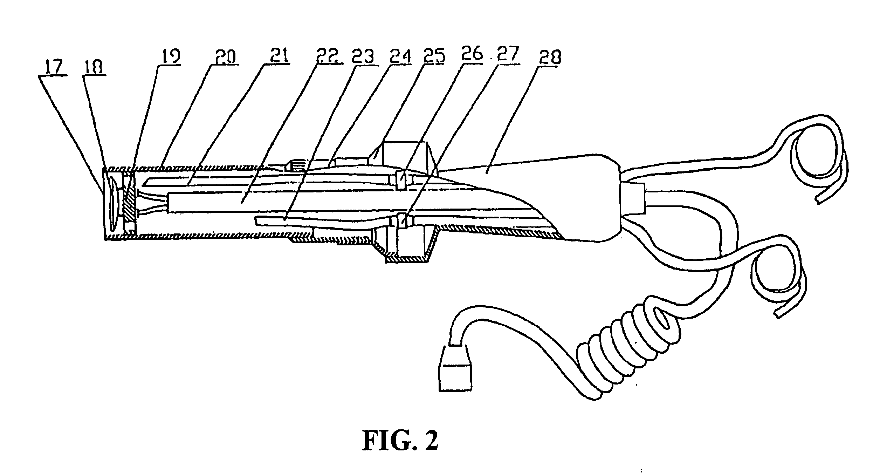

Referring to FIG. 2, the ultrasound applicator 16, which can be held by a hand, includes a handle comprising a shell and a lumen. The ultrasound transducer and the focusing means are placed in the lumen. The ultrasound applicator, which can be held by a hand, transmits high-frequency focused ultrasound. The ultrasound transducer converts the electric power into sound power. In order to relieve the heating of ultrasound applicator itself during the conversion, a cooling means is adopted. Water is used as a cooling medium in the present invention. The cooling water is led from the inlet water pipe 23 and drained through the outlet water pipe 21. The inlet water pipe 23 and the outlet water pipe 21 are respectively connected through the quick coupler 26, 27 within the handle sleeve 28 and then they are led out of the ultrasound applicator. In order to prevent air bubbles in common water from expanding due to the ultrasound action, distilled water is preferably used as the cooling medium. The quick coupler 26, 27 are fastened on the handle 25. Two holes on sides of the handle 25 are configured to install the quick coupler 26, 27. The power supply is connected to the ultrasound applicator through the cable 22 which is a standard coaxial-cable or cable with two cores. Two electrodes of the cable are respectively jointed with two electrodes of the ultrasound transducer by welding or through coaxial threaded couplings. The cable 22 goes through the lumen of the handle sleeve 28 and goes out from the hole on the front distal of the handle 25. A protective cover for the cable at outlet is provided in order to prevent the cable from being damaged. The ultrasound transducer 18 is supported by two metal electrodes, which are connected to the fixing plate 19. The fixing plate 19 is fixed with the cable 22. There is a space left between the fixing plate 19 and the outer sleeve 20. The acoustic membrane 17 is fixed at the front end of the outer sleeve 20 by means of adhesive joining. The outer sleeve 20 is connected to the adjusting focusing means 24 by threaded coupling. The outer sleeve 20 is detachable at any time. The adjusting focusing means 24 is connected to the handle 25 by threaded coupling. The adjusting focusing means 24 and the handle 25 are rotatable with each other. The handle sleeve 28 is connected to the handle 25 by threaded coupling firmly. Within the outer sleeve 20, an acoustic membrane 17 is placed in front of the ultrasound transducer 18 and fixed at the front distal of the ultrasound therapeutic applicator 16. The ultrasound transducer 18 in front of fixing plate 19 is connected to the panel 7 through the cable 22. The outlet water pipe 21 is placed on one side of the cable 22 and the inlet water pipe 23 is placed on the other side of the cable 22. The adjusting focusing means 24 is connected to the handle 25 by threaded coupling. The inlet water pipe 23 and the outlet water pipe 21 are respectively connected through the quick coupler 26, 27. The inlet water pipe 23 is connected to the water outlet 13 of cooling water tank and the outlet water pipe 21 is connected to the water inlet 11 of cooling water tank.

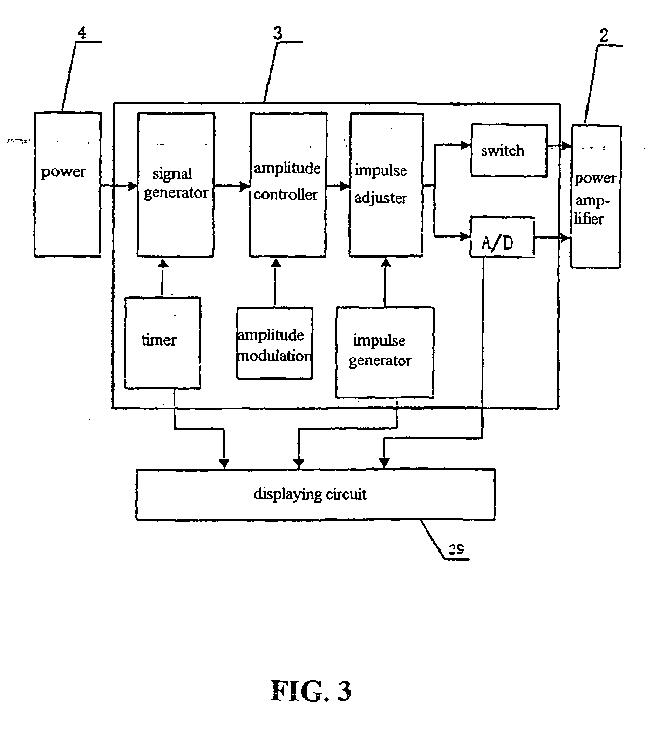

FIG. 3 is a block diagram of the controlling device according to the invention. The controlling device includes the controller 3, the power supply 4, the power amplifier 2 and the display circuit in the panel 7. The controller 3 includes a singlechip(optional for type 89C51), a controlling switch and an A/D convertor (optional for type ADC0809). The displaying circuit includes a display driver (optional for type 82C79) and a nixie tube connected to the LED display. An amplitude controller is respectively connected to a signal generator, an amplitude modulator and a pulse modulator. The pulse modulator is connected to the pulse generator and the controller's switch (optional for relay) and A/D convertor. The pulse generator is respectively connected to the pulse modulator and the displaying circuit. A timer is connected to the display circuit. Both the controller's switch and the A/D convertor are connected to power amplifier 2. The power amplifier 2 is connected to the output. The controlling functions are programmed into the singlechip that controls the ultrasound therapeutic equipment, including timing, starting, stopping and etc. The standard power amplifier sold in market is adopted, for example, VDS series products manufactured in the USA.

The power supply 4 consists of a rectifier diode and a voltage regulator. As showed in FIG. 4 and FIG. 5, the power supply 4 provides the power to each component and one circuit is available for the water pump. FIG. 4 is the circuit diagram of the controlling device. S1, S2, S3 are connected to the secondary windings of the transformer. S6, S7 are connected to the power amplifier 2. S8, S9, S10 are connected to the controller 3.

FIG. 5 shows the circuit to supply power to the water pump. S4, S5 are connected to the secondary windings of the transformer, which is separated from the secondary windings connected to S1, S2 and S3. S11, S12 are connected to the water pump.

The circuit boards in the above invention embodiments are installed in the controlling device cabinet as shown in FIG. 1. The technical engineers can make changes according to FIG. 3 to achieve the same function.

## Claims

1. An ultrasound therapeutic equipment, comprising:
an ultrasound transducer (18) transmitting a therapeutic ultrasound wave to a target region,
a focusing means (24) for focusing the therapeutic ultrasound wave to the target region, and
a hand-held ultrasound applicator (16), the ultrasound transducer (18) and the focussing means (24) being placed in the ultrasound applicator (16), and
a controlling device (8) for controlling the whole system of the ultrasound therapeutic equipment,
**characterized by**
cooling means including an external water tank (14) connected with an inlet water pipe (23) and an outlet water pipe (21),
ultrasound applicator (16) comprising a outer sleeve (20), a fixing plate (19), and an acoustic membrane (17), and a cable (22), wherein
the fixing plate (19) is held between inner walls of the outer sleeve (20),
the acoustic membrane (17) is fixed at a frontend of the outer sleeve (20),
the ultrasound transducer (18) is fixed to the fixing plate (19) and is positioned between the fixing plate (19) and the acoustic membrane (17),
the cable (22) is a standard coaxial-cable or cable with two cores and two electrodes of the cable (22) are respectively jointed with two electrodes of the ultrasound transducer (18),
the ultrasound transducer (18) is supported by two metal electrodes, which are connected to the fixing plate (19) and the fixing plate (19) is fixed with the cable (22), wherein a space is left between the fixing plate (19) and the outer sleeve (20) so that the space left between the fixing plate (19) and the outer sleeve (20) allows water to flow there through to and from the ultrasound transducer (18), and
the outlet water pipe (21) is placed on one side of the cable (22) and the inlet water pipe (23) is placed on the other side of the cable (22) so that the outlet water pipe (21) is aligned with its end to a first portion of the space between the fixing plate (19) and the outer sleeve (20) and the inlet water pipe (23) is aligned with its end to a second portion of the space between the fixing plate (19) and the outer sleeve (20).

2. An equipment as defined in claim 1, wherein said controlling device has a frequency width in the range of from 5 MHz to 25 MHz.

3. An equipment as defined in claim 2, wherein said controlling device has a frequency width in the range of from 9 MHz to 11 MHz.

4. An equipment as defined in claim 1, wherein the focal length of the ultrasound applicator ranges from 5 mm to 50 mm.

5. An equipment as defined in claim 4, wherein the focal length of the ultrasound applicator ranges from 9 mm to 11 mm.

6. An equipment as defined in claim 1, wherein said water piping includes an inlet water pipe (23) and an outlet water pipe (21), the inlet water pipe (23) and outlet water pipe (21) are connected in a loop through said sealed lumen of ultrasound applicator.

7. An equipment as defined in claim 1, wherein the front distal of said ultrasound applicator has a diameter in the range of from 5 mm to 80 mm.

8. An equipment as defined in claim 7, wherein the front distal of the said ultrasound applicator has a diameter in the range of from 14 mm to 16 mm.

9. An equipment as defined in claim 1, wherein said ultrasound transducer (18) is detachable and installed within the handheld ultrasound applicator.

10. An equipment as defined in claim 7, wherein said ultrasound transducer (18) is detachable and installed within the handheld ultrasound applicator.

11. An equipment as defined in claim 8, wherein the said ultrasound transducer (18) is detachable and installed within the handheld ultrasound applicator.

12. The equipment of claim 1 wherein the water contained in the said cooling means is the purified water.

## Patentansprüche

1. Ultraschallbehandlungsapparatur, umfassend:
einen Ultraschallwandler (18), der eine Behandlungsultraschallwelle zu einem Zielbereich sendet,
ein Fokussiermittel (24) zum Fokussieren der Behandlungsultraschallwelle auf den Zielbereich, und
einen Ultraschallhandapplikator (16), wobei der Ultraschallwandler (18) und das Fokussiermittel (24) in dem Ultraschallapplikator (16) angeordnet sind, und
eine Steuereinrichtung (8) zum Steuern des gesamten Systems der Ultraschallbehandlungsapparatur,
**gekennzeichnet durch**
ein Kühlmittel, das einen mit einem Einlasswasserrohr (23) und einem Auslasswasserrohr (21) verbundenen Außenwassertank (14) enthält, einen Ultraschallapplikator (16), der eine äußere Hülle (20),
eine Fixierplatte (19) und eine Schallmembran (17) umfasst, und ein Kabel (22), wobei
die Fixierplatte (19) zwischen Innenwänden der äußeren Hülle (20) gehalten ist,
die Schallmembran (17) an einem Vorderende der äußeren Hülle (20) fixiert ist,
der Ultraschallwandler (18) an der Fixierplatte (19) fixiert und zwischen der Fixierplatte (19) und der Schallmembran (17) positioniert ist,
das Kabel (22) ein standardmäßiges Koaxialkabel oder ein Kabel mit zwei Adern ist und zwei Elektroden des Kabels (22) jeweils mit zwei Elektroden des Ultraschallwandlers (18) verbunden sind,
der Ultraschallwandler (18) von zwei Metallelektroden getragen ist, die mit der Fixierplatte (19) verbunden sind, und die Fixierplatte (19) an dem Kabel (22) fixiert ist, wobei zwischen der Fixierplatte (19) und
der äußeren Hülle (20) ein Raum freigelassen ist, so dass der zwischen der Fixierplatte (19) und der äußeren Hülle (20) freigelassene Raum den Durchfluss von Wasser zu und von dem Ultraschallwandler (18) gestattet, und
das Auslasswasserrohr (21) an einer Seite des Kabels (22) und das Einlasswasserrohr (23) an der anderen Seite des Kabels (22) derart angeordnet ist, dass das Auslasswasserrohr (21) mit seinem Ende mit einem ersten Teil des Raums zwischen der Fixierplatte (19) und der äußeren Hülle (20) und das Einlasswasserrohr (23) mit seinem Ende mit einem zweiten Teil des Raums zwischen der Fixierplatte (19) und der äußeren Hülle (20) ausgerichtet ist.

2. Apparatur nach Anspruch 1, wobei die Steuereinrichtung eine Frequenzbreite im Bereich von 5 MHz bis 25 MHz aufweist.

3. Apparatur nach Anspruch 2, wobei die Steuereinrichtung eine Frequenzbreite im Bereich von 9 MHz bis 11 MHz aufweist.

4. Apparatur nach Anspruch 1, wobei die Brennweite des Ultraschallapplikators im Bereich von 5 mm bis 50 mm liegt.

5. Apparatur nach Anspruch 4, wobei die Brennweite des Ultraschallapplikators im Bereich von 9 mm bis 11 mm liegt.

6. Apparatur nach Anspruch 1, wobei die Wasserrohre ein Einlasswasserrohr (23) und ein Auslasswasserrohr (21) enthalten, wobei das Einlasswasserrohr (23) und das Auslasswasserrohr (21) in einem Kreis durch den versiegelten Lumen des Ultraschallapplikators verbunden sind.

7. Apparatur nach Anspruch 1, wobei das vordere, distale Ende des Ultraschallapplikators einen Durchmesser im Bereich von 5 mm bis 80 mm aufweist.

8. Apparatur nach Anspruch 7, wobei das vordere, distale Ende des Ultraschallapplikators einen Durchmesser im Bereich von 14 mm bis 16 mm aufweist.

9. Apparatur nach Anspruch 1, wobei der Ultraschallwandler (18) abnehmbar und in dem Ultraschallhandapplikator eingebaut ist.

10. Apparatur nach Anspruch 7, wobei der Ultraschallwandler (18) abnehmbar und in dem Ultraschallhandapplikator eingebaut ist.

11. Apparatur nach Anspruch 8, wobei der Ultraschallwandler (18) abnehmbar und in dem Ultraschallhandapplikator eingebaut ist.

12. Apparatur nach Anspruch 1, wobei das in dem Kühlmittel enthaltene Wasser gereinigtes Wasser ist.

## Revendications

1. Un équipement thérapeutique aux ultrasons, comprenant :
- un transducteur aux ultrasons (18) qui transmet une onde à ultrason thérapeutique vers une zone cible,
- un moyen de focalisation (24) pour la focalisation de l'onde à ultrason thérapeutique vers la zone cible, et
- un applicateur à ultrason tenu à la main (16), le transducteur aux ultrasons (18) et le moyen de focalisation (24) étant placés dans l'applicateur à ultrason (16), et
- un dispositif de contrôle (8) pour le contrôle du système complet de l'équipement thérapeutique aux ultrasons,
**caractérisé :**
- **par** un moyen de refroidissement qui inclut un réservoir d'eau externe (14) relié à une conduite d'eau d'arrivée (23) et à une conduite d'eau de sortie (21), l'applicateur à ultrason (16) comprenant un manchon extérieur (20), une plaque de fixation (19) et une membrane acoustique (17) et un câble (22), dans lequel :
- en ce que la plaque de fixation (19) est maintenue entre des parois intérieures du manchon extérieur (20),
- en ce que la membrane acoustique (17) est fixée sur une extrémité avant du manchon extérieur (20),
- en ce que le transducteur aux ultrasons (18) est fixé sur la plaque de fixation (19) et est positionné entre la plaque de fixation (19) et la membrane acoustique (17),
- en ce que le câble (22) est un câble coaxial standard ou un câble avec deux torons et deux électrodes du câble (22) qui sont reliées respectivement à deux électrodes du transducteur aux ultrasons (18),
- en ce que le transducteur aux ultrasons (18) est soutenu par deux électrodes en métal qui sont connectées à la plaque de fixation (19) et en ce que la plaque de fixation (19) est fixée avec le câble (22), dans lequel un espace est laissé entre la plaque de fixation (19) et le manchon extérieur (20) de telle sorte que l'espace laissé entre la plaque de fixation (19) et le manchon extérieur (20) permet à l'eau de couler à travers celui-ci vers et depuis le transducteur aux ultrasons (18), et en ce que la conduite d'eau de sortie (21) est placée sur un côté du câble (22) et la conduite d'eau d'arrivée (23) est placée sur l'autre côté du câble (22) de telle sorte que la conduite d'eau de sortie (21) est en alignement avec son extrémité par rapport à une première partie de l'espace entre la plaque de fixation (19) et le manchon extérieur (20) et la conduite d'eau d'arrivée (23) est en alignement avec son extrémité par rapport à une seconde partie de l'espace entre la plaque de fixation (19) et le manchon extérieur (20).

2. Un équipement tel qu'il est défini dans la revendication 1, dans lequel ledit dispositif de contrôle a une largeur de fréquence dans la plage de 5 MHz à 25 MHz.

3. Un équipement tel qu'il est défini dans la revendication 2, dans lequel ledit dispositif de contrôle a une largeur de fréquence dans la plage de 9 MHz à 11 MHz.

4. Un équipement tel qu'il est défini dans la revendication 2, dans lequel la longueur de focalisation de l'applicateur à ultrason s'étend de 5 mm à 50 mm.

5. Un équipement tel qu'il est défini dans la revendication 4, dans lequel la longueur de focalisation de l'applicateur à ultrason s'étend de 9 mm à 11 mm.

6. Un équipement tel qu'il est défini dans la revendication 1, dans lequel ladite conduite d'eau inclut une conduite d'eau d'arrivée (23) et une conduite d'eau de sortie (21) et dans lequel la conduite d'eau d'arrivée (23) et la conduite d'eau de sortie (21) sont reliées dans une boucle à travers ledit lumen étanche de l'applicateur à ultrason.

7. Un équipement tel qu'il est défini dans la revendication 1, dans lequel la partie distale avant dudit applicateur à ultrason a un diamètre dans la plage qui s'étend de 5 mm à 80 mm.

8. Un équipement tel qu'il est défini dans la revendication 7, dans lequel la partie distale avant dudit applicateur à ultrason a un diamètre dans la plage qui s'étend de 14 mm à 16 mm.

9. Un équipement tel qu'il est défini dans la revendication 1, dans lequel ledit transducteur aux ultrasons (18) est amovible et est installé à l'intérieur de l'applicateur à ultrason tenu à la main.

10. Un équipement tel qu'il est défini dans la revendication 7, dans lequel ledit transducteur aux ultrasons (18) est amovible et est installé à l'intérieur de l'applicateur à ultrason tenu à la main.

11. Un équipement tel qu'il est défini dans la revendication 8, dans lequel ledit transducteur aux ultrasons (18) est amovible et est installé à l'intérieur de l'applicateur à ultrason tenu à la main.

12. L'équipement selon la revendication 1, dans lequel l'eau contenue dans ledit moyen de refroidissement est de l'eau purifiée.
